## Europäisches Patentamt
## European Patent Office
## Office européen des brevets

(11) Publication number: **0 265 384**
**A2**

(12) **EUROPEAN PATENT APPLICATION**

(21) Application number: **87810591.5**

(22) Date of filing: **15.10.87**

(51) Int. Cl.⁴: **C 12 P 21/00**
**A 61 K 39/395, G 01 N 33/577**

(30) Priority: **17.10.86 GB 8624899**

(43) Date of publication of application:
**27.04.88 Bulletin 88/17**

(84) Designated Contracting States:
**AT BE CH DE ES FR GB GR IT LI LU NL SE**

(71) Applicant: **SANDOZ AG**
**Lichtstrasse 35**
**CH-4002 Basel (CH)**

(84) Designated Contracting States:
**BE CH ES FR GB GR IT LI LU NL SE**

(71) Applicant: **SANDOZ-PATENT-GMBH**
**Humboldtstrasse 3**
**D-7850 Lörrach (DE)**

(84) Designated Contracting States: **DE**

(71) Applicant: **SANDOZ-ERFINDUNGEN**
**Verwaltungsgesellschaft m.b.H.**
**Brunner Strasse 59**
**A-1235 Wien (AT)**

(84) Designated Contracting States: **AT**

(72) Inventor: **Di Padova, Franco**
**Wartenbergstrasse 52**
**CH-4127 Birsfelden (CH)**

**Zenke, Gerhard**
**In den Burgreben 3 Herten**
**D-7888 Rheinfelden (DE)**

The applicant has filed a statement in accordance with Rule 28 (4) EPC (issue of a sample only to an expert). Accession number(s) of the deposit(s): 86100201, 86100202, 86100203, 86100204.
Claims for the following Contracting States: ES + GR.

(54) **Monoclonal antibodies to a colony stimulating factor.**

(57) A class of novel monoclonal antibodies specific for or directed to epitopes on primate granulocyte-macrophage colony-stimulating factor are disclosed. They are suitable for use in diagnostic/assay kits, in affinity purification of GM-CSF from biological fluids or tissue, and therapeutically.

EP 0 265 384 A2

Bundesdruckerei Berlin

Description

# MONOCLONAL ANTIBODIES TO A COLONY-STIMULATING FACTOR

TECHNICAL FIELD

The invention relates to monoclonal antibodies to primate granulocyte-macrophage colony-stimulating factor (primate GM-CSF), a method of producing such antibodies, hybridoma cells capable of producing the antibodies and uses of the antibodies.

BACKGROUND ART

Polyclonal antibodies, e.g. antisera, to colony-stimulating factors are disclosed or alluded to e.g. in the following publications:
-R.K. Shadduck & A.Waheed, Blood Cells 5 (1979) 421-434;
- A. Waheed & R.K. Shadduck, Blood 60 (1982) 238-244;
- J.E. Straneva et al., Progr. Clin. Biol. Res. 215 (1986) 253-7;
- V. Barak et al., J. Reproductive Immunology 9 (1986) 355-363;
- K.A. Foon et al., Blood 64 (1984) 1085-1093.
  Polyclonal antibodies, e.g. antisera, to GM-CSF are disclosed or alluded to in the following publications:
- R.A. Donahue et al., Nature 321 (June 26, 1986) 872-875;
- J.F. DeLamarter et al., EMBO J. 4 (1985) 2575-2581;
- Y.C. Chang et al., Cell 47 (October 10, 1986) 3-10;
- D.Y. Mochizuki et al., J. Immunol. 136 (1986) 3706-3710.
  Monoclonal antibodies to colony-stimulating factors which are either ill-defined or other than GM-CSF are disclosed or alluded to in the following publications:
- C.J. Paige et al., Eur. J. Immunol. 14 (1984) 979-987;
- R.K. Shadduck et al., Exp. Hematol. 14 (1986) 812-818;
- T. Kawaguchi et al., Blood 67 (June 1986) 1619-1623;
- Chugai EP 225 583 (published June 16, 1987);
- Kirin-Amgen EP 227 367 (published July 1, 1987).
  Monoclonal antibodies to GM-CSF are envisaged or alluded to in the following publications:
- A. Miyajima et al., EMBO J. 5 (June 1986) 1193-1197;
- Sandoz WO 86/00639 (published January 30, 1986);
- D.C. Young et al., J. Clin. Invest. 79 (January 1987) 100-106.
  None of the above publications, however, is specific or enabling as to monoclonal antibodies to primate GM-CSF.

DISCLOSURE OF THE INVENTION

The present invention provides monoclonal antibodies to primate GM-CSF, in particular to human GM-CSF or recombinant primate GM-CSF, more especially to human recombinant GM-CSF.

Primate GM-CSF is herein defined as any GM-CSF which is endogenous to a primate species, and recombinant variants thereof.

The full amino acid sequences of two human allelic GM-CSFs and one gibbon GM-CSF are disclosed e.g. in WO 86/00639 and in other publications.

Primate GM-CSF is for example the complete proteins, or the mature protein part of the proteins in Figure 1 of WO 86/00639, and allelic or recombinant variants thereof. In that publication the sequences for gibbon GM-CSF and for two human allelic GM-CSFs differing in having either Ile or Thr at amino acid position No. 100 from the beginning of the mature protein, namely for GM-CSF-Ile and, respectively, GM-CSF-Thr, are disclosed.

Human GM-CSF is for example the complete human proteins, or the mature protein part of the human proteins in Figure 1 of WO 86/00639, namely GM-CSF-Ile and, respectively, GM-CSF-Thr, and allelic or recombinant variants thereof.

Allelic variants are proteins differing from the above defined proteins in having one or at most a few, e.g. two to five amino acids different from the amino acids in the above-defined proteins and still having immunological cross-reactivity with the monoclonal antibodies of the invention.

Recombinant variants are e.g. synthetic proteins as defined in Cetus USP 4 530 787, particularly synthetic proteins as defined on column 2 lines 40 to 53, or muteins as defined on column 3 lines 1 to 9 of the above USP; or recombinant variants are e.g. variants as disclosed in Hoechst EP 228 018.

Preferred are monoclonal antibodies to human GM-CSF.

The term "antibody" as used in this invention is meant to include intact molecules as well as fragments thereof, such as, for example, Fab and F(ab')$_2$, which are capable of binding the epitopic determinant.

The monoclonal antibodies of the invention are suitable for use in affinity purification of GM-CSF from biological fluids or tissue, in diagnostic/assay kits, and therapeutically, particularly the monoclonal antibody produced by cell-line 1-3-2 more particularly identified below.

They allow:

a) affinity purification of GM-CSF to more than 98% purity with a recovery of 52-96% using a single step chromatography, on an industrial scale, the single step resulting in an enrichment by a factor of the order of several hundreds,

b) quantitative monitoring of GM-CSF, and

c) functional inactivation of GM-CSF and are thus suitable for use in the prophylaxis and treatment of conditions resulting from excessive GM-CSF activity, e.g. in myeloid leukemia.

The monoclonal antibodies of the invention may recognize epitopes situated anywhere on the protein part of the GM-CSF molecule. The monoclonal antibodies produced may cross-react with the GM-CSFs of different primate species. They may also be species specific. They may recognize e.g. an epitope situated at a place other than on the fist 16 N-terminal amino acids of mature human GM-CSF.

It is to be appreciated that GM-CSF may vary as to glycosylation and in other respects, it may e.g. be unglycosylated, or a mixture of several forms with various degrees of glycosylation. Thus GM-CSFs of all size classes with various degrees of glycosylation, e.g. individually or as a mixture, may be used, such as recombinant GM-CSF, microbially expressed or expressed in eukaryotic cells.

In a further aspect the invention provides a process for the production of a monoclonal antibody to primate GM-CSF. This may be effected by essentially standard techniques, e.g. via a stepwise procedure comprising:

1. immunization by administration of an immunogenic GM-CSF preparation to an appropriate animal species;

2. recovery of antibody-producing, e.g. spleen or lymph-node, cells sensitized to the antigen;

3. immortalization of recovered cells, e.g. by fusion with an appropriate myeloma cell line, to produce hybridoma cell lines;

4. selection of an immortalized cell clone, e.g. hybridoma line, producing monoclonal antibodies as required; and

5. recovery of the monoclonal antibodies produced by the cell line.

Primate GM-CSF, preferably in purified form, may be employed as immunogen. Purification may be effected e.g. as described in Science 228 (1985) 810-815 or Cancer Cells 3/Growth Factors and Transformation (1985) 235-242. The antibody-producing cells used are preferably spleen cells or lymph node cells. The particular animal species from which the myeloma and antibody-producing cells are derived is not critical insofar as it is possible to fuse the cells of the one species with another, e.g. mouse to rat, rat to human, or mouse to human. It is preferred, however, to use the same species of animal as a source of both myeloma and antibody-producing cells. One preferred cell line for the practice of this invention is a fused cell hybrid between an antigen-primed mouse spleen cell and a mouse myeloma cell. The hybridomas resulting from the fusion are sytematically examined for production of antibodies which selectively react with primate GM-CSF. It should be noted that monoclonal antibodies raised against a single antigen may be distinct from each other depending on the determinant that induced their formation; but for any given hybridoma (clone), all of the antibodies it produces are monospecific for a particular antigenic determinant in the GM-CSF molecule.

Step 1. (immunization) is suitably carried out using rats or mice, e.g. female Balb/c mice as recipient. Any combination of subcutaneous, intramuscular, intraperitoneal or intravenous injections of buffered GM-CSF solution in the presence of a suitable adjuvant may be used. The immunization scheme and the concentration of GM-CSF should be selected such that adequate amounts of antigen-stimulated lymphocytes are formed. For example, administration of the immunogen GM-CSF is by s.c. or i.p. injection in an amount of from ca.50 to 200, e.g. ca. 100 microg followed by booster injections, i.p., s.c. or i.m., 3 to 4 weeks later. Animals may be selected for further use on the basis of measured antisera employing critera such as
- high titre in an indirect enzyme-linked immunosorbent assay (ELISA) and/or radioimmunoassay (RIA),
- relative avidity for GM-CSF determined in competitive indirect ELISA, and/or
- isotype distribution.

Animals selected are given further booster injections, e.g. in accordance with the specific procedures hereinafter described in Example 1.

The antigen is primate GM-CSF, preferably in purified form.

In step 2. (recovery), antibody-producing cells are collected and a cell suspension in a suitable medium is prepared.

The antibody-producing cells may be obtained from any site of an individual host, typically from the spleen, lymph nodes, peripheral blood or appropriate combinations thereof; spleen or lymph node cells are most commonly used.

After final immunization, a site containing antibody-producing cells, e.g. the spleen, is extracted from mice which have attained the desired antibody titer and spleen cells are prepared.

Step 3. (immortalization) may be performed in accordance with any of the techniques practiced in the art, e.g. using the method described by S. Fazekas et al., J. Immunol. Methods 35, 1-32 (1980), the myeloma (or plasmacytoma) cell line used preferably being non-producing, adherently-growing, and of modest growth requirements. A preferred myeloma line is a mouse (Balb/c) non-producing line, e.g. Sp 2/0 - Ag 14 (Nature 276 [1978] 269-270) or P3-X63-Ag8-653 (J. Immunol. 123 [1979] 1548-1550) or a variant thereof. A suitable fusion promoter, e.g. polyethylene glycol, is normally used. A ratio of about 10 spleen cells or lymph cells per myeloma cell is preferred. A total volume of about 1 ml of fusion medium is adequate for $10^8$ spleen cells or lymph node cells. The myeloma cell line used should preferably be of the so-called "drug resistant" type, so that, in a selective medium, unfused myeloma cells die whilst hybrids survive. Cell lines resistant to 8-azaguanine, which lack the enzyme hypoxanthine-guanine phosphoribosyl-transferase and which therefore cannot grow in a HAT medium (hypoxanthine, aminopterin, thymidine), are most frequently used. The unfused spleen cells or lymph node cells, the unfused myeloma cells and the fused cells are diluted and cultivated in a selective medium in which the unfused myeloma cells do not divide so that the unfused cells die (about 1-2 weeks). The individual fused cells are isolated by adjusting the volume of the diluent so that a given number of cells (about 1 to 4) is placed in each individual vessel (for example each well of a microtitre plate). The medium (for example HAT medium) prevents the growth of the resistant (for example against 8-azaguanine) unfused myeloma cell line, and thus it dies. The unfused spleen cells or lymph node cells have only a limited number of division cycles and hence these cells also die after a certain period (about 1-2 weeks). In contrast, the fused cells continue to divide since they have inherited permanent growth from the parent myeloma cells and the ability to synthesize e.g. the enzyme hypoxanthine-guanine phosphoribosyltransferase from the parent spleen cells or lymph node cells, and thus they are able to survive in the selective medium.

In step 4. (selection), growing hybridoma lines are screened for antibody production against primate GM-CSF, e.g. in a regular ELISA system, e.g. as hereinafter described in Example 1. It is desirable to use test conditions such that very early characterization of hybridoma clones is possible, e.g. by using a convenient test system such as ELISA, and a sensitive detection method, e.g. with two amplification steps, in order to assay possibly low concentrations of monoclonal antibodies present in hybridoma supernatants. The selected hybridoma cell lines are thereafter perpetuated by cloning in accordance with known procedures to achieve large-scale production of the monoclonal antibody.

The suspension is distributed into wells on an incubation plate and cultivated in a $CO_2$ incubator at 35-40°C for 10-30 hours. In a subsequent period of 1-3 days, half the volume of the supernatant of the culture is discarded every 10-30 hours and an equal volume of a fresh HAT medium or any other appropriate medium is added. Thereafter, cultivation is effected in a $CO_2$ incubator at 35-40°C for 10-14 days.

The myeloma cells used are e.g. 8-azaguanine resistant and hybridomas obtained by fusing two of them with each other are incapable of surviving in a hypoxanthine-aminopterin-thymidine containing medium (HAT medium). However, hybridomas obtained by fusing the antibody-producing cells with themselves or with myeloma cells are capable of surviving in the HAT medium. In addition, the life of the hybridomas of two antibody-producing cells is limited. Therefore, screening for the desired hybridomas of myeloma and antibody-producing cells can be accomplished by cultivation in a HAT medium.

After identifying the wells containing colonies of the intended hybridomas half the volume of the supernatant of the culture is discarded and an equal volume of an HT medium (aminopterin-free HAT medium) is added and transfer into an HT medium is repeated every 10-30 hours in a subsequent period of 1-3 days.

After conducting cultivation in the HT medium for 3-4 days, part of the supernatant of the culture is sampled for measuring the antibody titer by an appropriate method such as ELISA.

Other cell lines may be employed in accordance with the particular hybridoma to be selected and the composition of the media to be employed is then varied accordingly.

The hybridomas which have been found to have the ability to specifically produce the intended antibody are transferred onto another plate for achieving their cloning. Cloning can be performed by various methods such as: limiting dilution wherein dilution is effected in such a manner that one hybridoma is plated in one well; the soft agar method wherein colonies are taken by plating on a soft agar medium; the use of a micro-manipulator for delivering and plating one cell at a time; and sorter cloning which employs a cell sorter to separate one cell at a time. Limiting dilution is a simple method and commonly used.

Cloning is repeated two to four times by an appropriate method such as limiting dilution for the wells which have been found to have a predetermined antibody titer and those hybridomas which have been found to have that titer in each run are selected as the monoclonal antibody-producing hybridoma cells.

Step 5. (recovery) is effected in known manner. The cloned hybridomas are e.g. cultured on a normal medium which has been changed from the HT medium. Large-scale cultivation is effected with a whirler or spinner employing a large incubation bottle. The resulting supernatant is subjected to gel filtration and other appropriate treatments for collecting IgG fractions, which are then purified to obtain anti-GM-CSF monoclonal antibodies.

The hybridomas can also proliferate in the abdominal cavity of a mouse of the same strain as used in the preparation of the antibody-producing cells (e.g. BALB/c mouse) or a Nu/Nu mouse. For instance, 8 to 10-week old BALB/c female mice are injected intraperitoneally with $2\times10^6$ to $4\times10^6$ per animal of the anti-GM-CSF monoclonal antibody-producing hybridoma cells; in 10-21 days, the hybridomas grow into ascitic cancer; a sample of ascites is taken from each mouse and freed of any solids content by centrifugation; the resulting product can be used as a monoclonal antibody in such applications as the purification and assaying of primate GM-CSF.

If further purification is necessary, the supernatant obtained by centrifugation may be passed at least once through a column packed with a suitable adsorbent such as DEAE-Sepharose or protein A-Sepharose, followed by collection of Ig fractions.

The monoclonal antibodies obtained are classified, or their isotypes and subclasses are determined, by such methods as the Ouchterlony method, ELISA and RIA. The Ouchterlony method is simple but requires enrichment of the monoclonal antibodies if their concentration is low. In ELISA or RIA, the supernatant of the culture may be directly reacted with an antigen-adsorbed solid phase and antibodies corresponding to individual IgG subclasses can be used as the secondary antibody.

Protein contents may be determined by the Folin-Lowry method and on the basis of absorbance at 280 nm [$OD_{280}$ = 1.4 corresponds to 1 microg of immunoglobulin per ml].

By application of the above procedures it is possible to select monoclonal antibodies which exhibit various degrees of specificity and affinity for the "target" GM-CSF.

The present invention permits the ready obtention of monoclonal antibodies of especially the class IgG, e.g. of the sub-cass $IgG_1$. Insofar as such antibodies are especially suited to use in diagnostic/assay kits, or for affinity purification, e.g. in view of their sensitivity to changes in pH or molarity of the buffer, or for functional inactivation, these are preferred.

Monoclonal antibodies as described above, as well as hybridoma lines producing them are entirely novel and, as will be appreciated from the foregoing description of their general and specific properties, well adapted for use in diagnostic/assay kit systems, e.g. for monitoring GM-CSF levels in plasma, blood or other biological fluids or tissues, e.g. cell culture supernatants, or for affinity purification, or for functional inactivation. The present invention especially provides:

1) Three monoclonal antibodies of $IgG_{2b}$ and $IgG_1$ subclass that are particularly suitable for affinity purification since their relative affinities for GM-CSF are sensitive to changes in pH of the buffer system. These are produced by cell lines 5-2-4, 1-113-4 and 1-507-6, respectively, as described in Example 1, and the cell lines have been deposited on October 2, 1986 with the European Collection of Animal Cell Cultures (ECACC), Porton Down, Salisbury, Whiltshire SP4 0JG, United Kingdom, under Accession Numbers 86100202, 86100203 and, respectively, 86100204.

2) One monoclonal antibody of $IgG_1$ subclass that is particularly suitable for use in diagnostics and in drug monitoring since it can be used in an inhibition ELISA test with a sensitivity in the nanogram range. This is produced by cell-line 1-3-2, as described in Example 2, and the cell line has been deposited on October 2, 1986 with the ECACC under Acession Number 86100201.

3) Two monoclonal antibodies of $IgG_1$ and $IgG_{2b}$ subclass that are particularly suitable for functional inactivation of primate GM-CSF, e.g. in therapeutic treatment of acute or chronic myeloid leukemia or other diseases involving autocrine production or overproduction of GM-CSF. These are produced by deposited cell-lines 5-2-4 and, respectively, 1-3-2 mentioned above.

The present invention thus makes it possible, for the first time, to obtain monoclonal antibodies capable of being used in:

a) a system for the large-scale affinity purification of primate GM-CSF;

b) a system for quantitative monitoring of primate GM-CSF levels, e.g. in blood or cell culture supernatants, and

c) functional inactivation of primate GM-CSF, e.g., therapy.

Preferably the monoclonal antibodies described in Example 1 are used for a) and preferably the one described in Example 2 for b) and c).

The monoclonal antibodies of the invention react with GM-CSF in solution and GM-CSF bound to a solid phase. They recognize native GM-CSF. They have properties which make them useful in a surprisingly broad range of applications, in particular the antibodies produced by cell-line 1-3-2.

Optionally, a "cocktail" of antibodies may be used.

a) Affinity purification

The monoclonal antibodies of the invention are capable of being used in a system for the large-scale affinity purification of primate GM-CSF (as well as other hematopoietic growth factors which may share common or related epitopes).

Under "large-scale" system is to be understood a system for producing GM-CSF amounts larger than those usually required for normal research purposes, e.g. of more than about 10 mg, especially more than about 1 g.

Possible applications include the isolation of GM-CSF from natural or recombinant sources, the preparation of $^{35}$S-labelled GM-CSF, the study of the biological activity of specific size classes of GM-CSF, and industrial preparation of GM-CSF for pharmaceutical use.

For this use the monoclonal antibodies may be coupled to various support matrices such as CNBr-activated agarose (Sepharose ®), nonwoven fiber filters, etc. The source material is contacted with the immobilized antibody. Primate GM-CSF or antigenically related material binds to the antibody and is thereafter eluted from the immobilized antibody in a highly purified form.

The system comprises a support matrix and a monoclonal antibody to primate GM-CSF.

Crude cell culture supernatant such as supernatant obtained after culture of GM-CSF-transfected E.coli or CHO (Chinese Hamster Ovary) cells, or other sources of GM-CSF, may be used.

The GM-CSF concentration may e.g. be determined in an inhibition ELISA test sensitive in the nanogram range, e.g. using the monoclonal antibodies produced by cell line 1-3-2 described in Example 2.

The bound GM-CSF preferably is eluted from the complex with the antibody using a conventional solvent such as acetic acid, e.g. 0.1M at pH 2.8; glycine/HCl buffer, e.g. 0.1M at pH 2.5; sodium thiocyanate, e.g. 3M in PBS (phosphate-buffered saline) at pH 6.3; sodium acetate, e.g. 0.1M at pH 4.5; etc.

It may be desirable to work in an inert atmosphere such as nitrogen.

The resultant affinity-purified GM-CSF remains biologically active. Its purity is better than 98%. The recovery ranges from 52% to 96% after a single step affinity chromatography. Under near optimal conditions at least 1.2 mole GM-CSF are bound per mole of antibody, e.g. per mole of antibody produced by cell-line 5-2-4.

The only contaminant likely to remain after the single step chromatography is albumin, which may if desired be removed in a further step in known manner.

b) Quantitative monitoring

As mentioned above, the monoclonal antibodies of the invention are also capable of being used in a system for quantitative monitoring of levels of primate GM-CSF (as well as other hematopoietic growth factors which may share common or related epitopes), e.g. in blood or cell culture supernatants, in particular as components of diagnostic/assay kit systems, e.g. immuno assay kits.

Such assays depend on the specificity of an antigen-antibody reaction in which an antibody reacts selectively with the corresponding antigenic substance and they achieve such high levels of detection sensitivity that they are suitable for use in determining the antigenic substance and antibody titer.

Accordingly, in a yet further aspect the present invention provides an immuno assay kit or system, e.g. ELISA, RIA or fluorescence immuno-assay (FIA) kit or system, for GM-CSF monitoring, for example in subjects receiving GM-CSF therapy, said kit or system comprising:    A) a monoclonal antibody as defined above, and

   B) GM-CSF,

one of them being in the form of a labelled derivative, as components of said kit or system, appropriately together with further conventional components.

For example, primate GM-CSF may be assayed by ELISA as follows using the monoclonal antibodies of the present invention: the antibody is adsorbed onto a solid phase; that surface of the solid phase to which the antibody has not been adsorbed is covered with a protein that is not related to an antigen of interest; the surface of the solid phase is washed; an enzyme-conjugated antigen and a sample of interest are added to effect antigen-antibody reaction; an enzyme substrate is added and the decrease in absorbance following the addition of the sample is measured to determine the amount of the antigen.

More particularly, an inhibition ELISA may be performed in 5 steps as schematically shown below:

```
                    │
                   ╱│    Step      Step    Step        Step       Step
                  ╱ │
                 ╱  │    1         2b      3           4          5
          solid ╱   │
                ╱   │
        support ────│──GM-CSF ──mAb  — RAM IgG ──GAR*   +   S
               ╱    │              ↑
              ╱     │              │
             ╱      │              │
            ╱       │              │
                    │              │
                              mAb +
                              GM-CSF

                              Step
                              2a
```

mAb = monoclonal antibody
RAM IgG= rabbit anti-mouse immunoglobulin G
GAR * = goat anti-rabbit immunoglobulin G conjugated with alkaline phosphatase
S = substrate (p-nitro-phenylphosphate).

In Step 1 the ELISA plates are coated with the antigen GM-CSF.

In Step 2a the test samples containing different, unknown amounts of GM-CSF or known amounts of standard antigen are separately incubated with monoclonal antibody and transferred to the plates.

In Step 2b the free monoclonal antibodies bind to the antigen on the plate.

In Step 3 the amount of free antibody is determined by binding anti-antibody to it.

In Step 4 an anti-antibody enzyme conjugate is added

In Step 5 reaction with substrate allows visualization.

After spectrophotometric reading at $OD_{405}$ a standard curve is drawn and the amount of GM-CSF in the test samples determined.

The monoclonal antibody produced by cell-line 1-3-2 is especially preferred for the above indirect ELISA.

Depending i.a. on the affinity characteristics of the monoclonal antibodies of the invention, other set-ups, such as the following, may be used in order to determine GM-CSF levels in e.g. the serum of patients:

6

```
              Step          Step          Step      Step     Step
  solid       1             2             3         4        5
  support ──── mAb 1      ───GM-CSF     ───mAb 2-B   St*    + S
                           standard

                           or test
                           sample
```

mAb 1 = monoclonal antibody to primate GM-CSF of high affinity

mAb 2 = monoclonal antibody to primate GM-CSF of high affinity and recognizing an epitope different from the epitope recognized by mAb1

B = enzyme conjugated to mAb2, or biotin label, or some other suitable marker

St* = streptavidin conjugated with an appropriate enzyme (in the case of biotinylated mAb 2)

S = substrate

In Step 1 the ELISA plats are coated with mAb1.

In Step 2 the plates are incubated with GM-CSF of known concentration as standard or with test samples containing unknown amounts of GM-CSF.

In Step 3 mAb2 is used to determine the amount of solid-phase bound GM-CSF.

In Step 4 e.g. biotinylated mAb2 is detected with a streptavidin-enzyme conjugate.

In Step 5 reaction with substrate permits visualization.

Kits as defined above are useful for diagnostic purposes, e.g. for determining quantities of GM-CSF present in blood, blood plasma or urine, e.g. as a means of establishing an appropriate dosaging regimen for patients receiving GM-CSF therapy.

Kits, e.g. ELISA, RIA and FIA, kits in accordance with the invention may be of entirely conventional type for use in accordance with conventional RIA, ELISA and FIA assay techniques. Thus kits will suitably comprise in addition to antibody A) above, an appropriate component B) above, and C) GM-CSF standard.

Suitably component A) or B) will be tritiated. The standard C) will generally be a solution or the like comprising a known quantity of the GM-CSF to be assayed.

In use, e.g. lyophilised antibody is dissolved and incubated together with e.g. component B) and with either the sample to be assayed or component C). Incubation is preferably effected with cooling e.g. at 4°C. The pH of the incubating mixture is preferably kept in the range of from about 5 to 8, e.g. at about pH 7 or 8, preferably with the aid of a buffering agent such as a citrate or Tris buffer.

Incubation conveniently lasts for at least 2 hours, e.g. from about 6 to 12 hours. After incubation the fraction of e.g. component B) bound to the antibody is separated from the unbound fraction, e.g. by the use of a second antibody or any suitable procedure to separate the unbound fraction from the bound fraction. The amount of radioactivity in one fraction is then measured by standard techniques, e.g. by liquid scintillation counting after the addition of a secondary solute. The proportion of component B) bound to the antibody is inversely proportional to the amount of GM-CSF in the unknown plasma sample. For quantitative analysis it is usual to prepare a standard calibration curve by analysing solutions of GM-CSF of known concentration.

Assay kits/systems as defined above may be based on any of the conventional ELISA, RIA or FIA systems known in the art.

c) Functional inactivation

As mentioned above, the monoclonal antibodies of the invention are also capable of being used for functionally inactivating GM-CSF. They can thus be used in vivo to bind primate GM-CSF (as well as other hematopoietic growth factors which may share common or related epitopes), e.g. in therapy.

Preferred for therapeutic use are the antibodies produced by cell lines 1-3-2 and 5-2-4, especially 1-3-2.

GM-CSF is a growth factor for the leukemic cells from many myeloid leukemia patients, both chronic and acute, and several patients have been identified whose blast cells are autocrine for GM-CSF (S.C. Clark and R. Kamen, Science 236 [1987] 1229-1237), suggesting that within a population of leukemic cells there may be a selective advantage for cells expressing GM-CSF.

It appears thus advantageous in the treatment of such patients to deprive the leukemic cells of their selective advantage by inactivating GM-CSF via administration of corresponding monoclonal antibodies with neutralising activity.

The monoclonal antibodies of the invention are, therefore, also indicated for use as pharmaceuticals.

In particular, they are able to neutralise the biological activity of GM-CSF as measured in the chronic myeloid leukemia (CML) assay (J.D. Griffin et al., Blood 63 [1984] 904-911):

GM-CSF is able to stimulate peripheral blood cells from chronic myelogeneous patients to proliferate in vitro. After 3 days in culture, cell proliferation is quantitated by measuring 3H-thymidine incorporation into DNA. Half-maximal stimulation of CML cell proliferation occurs in the presence of 1 unit of GM-CSF. To identify monoclonal antibodies with neutralising ability, the purified monoclonal antibody to be tested is added to the CML cells with various concentrations of GM-CSF at the initiation of the cultures. A neutralising antibody is identified by its ability to inhibit GM-CSF-induced CML cell proliferation.

The monoclonal antibodies of the invention show this activity at concentrations of from about 1 ng to about 50 ng purified antibody, which inactivates about 0.03 ng GM-CSF protein (equivalent to about 1 unit activity in the CML assay).

The monoclonal antibodies to primate GM-CSF of the invention are, therefore, indicated for use in the treatment of conditions where a decrease of GM-CSF activity is desirable, such as acute or chronic myeloid leukemia and other diseases involving autocrine production or overproduction of GM-CSF.

For this indication the appropriate dosage will, of course, vary depending upon, for example, the monoclonal antibody employed, the host, the mode of administration and the nature and severity of the condition being treated. In larger mammals, for example humans, the appropriate daily dosage of monoclonal antibody may be conveniently administered, for example, in divided doses up to four times a day.

The dosage ranges for the administration of the monoclonal antibodies of the invention are those large enough to produce the desired effect in which the symptoms are ameliorated. The dosage should not be so large as to cause adverse side effects, such as unwanted cross-reactions, anaphylactic reactions, and the like. Generally, the dosage will vary with the age, condition, sex and extent of the disease in the patient and can be determined by one of skill in the art. The dosage can be adjusted individually in the event of any counter indications, immune tolerance or similar conditions.

The monoclonal antibodies of the invention may be administered by any conventional route for protein administration, in particular parenterally, e.g. in the form of injectable solutions or suspensions.

The monoclonal antibodies of the invention can be administered parenterally by injection or by gradual perfusion over time. The monoclonal antibodies of the invention can be administered intravenously, intraperitoneally, intramuscularly, subcutaneously, intracavity, or transdermally, alone or in combination with effector cells.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers, such as those based on Ringer's dextrose, and the like. Preservatives and other additives may also be present such as, for example, antimicrobials, anti-oxidants, chelating agents, and inert gases and the like.

The invention also relates to a method for preparing a medicament or pharmaceutical composition comprising the monoclonal antibodies of the invention, the medicament being used for therapy as indicated above.

The monoclonal antibody produced by cell-line 1-3-2 is the preferred compound for these indications.

The present invention also provides pharmaceutical compositions comprising a monoclonal antibody to primate GM-CSF in association with at least one pharmaceutical carrier or diluent. Such compositions may be manufactured in conventional manner.

The functional inactivation may further be used to eliminate the effect of GM-CSF in assay systems, which may mask the effect of other hematopoietic factors. The monoclonal antibodies of the invention may thus also find an indirect application in assay systems for such other hematopoietic factors, e.g. IL-3.

It will be appreciated that by application of the techniques of the present invention as generally taught herein, and proceeding e.g. analogously to the general methods of the Examples, hybridoma lines/monoclonal antibodies may readily be prepared which, though not identical with the specific product hybridoma lines/monoclonal antibodies of the Examples, will meet the same essential criteria, e.g. exhibit equivalent or even improved characteristics to those described above, in particular:
- specificity for protein determinants
- reasonable affinity and
- good antibody production.

The following Examples are illustrative of the invention. All temperatures are in degrees Centigrade.

Example 1: Preparation of monoclonal antibodies for affinity purification of GM-CSF

1) Immunization of mice [step 1.]

Mice (6 Balb/c) each receive 60 microg of GM-CSF-Thr conventionally purified from culture supernatant of Chinese Hamster ovary (CHO) cells transfected with the human GM-CSF-Thr gene. The GM-CSF is dissolved in 200 microl of phosphate buffered saline/Freund adjuvant 1:1. The immunization is effected as described by Stähli et al. in J. Immunol. Meth. 32 [1980] 297-304. The first administration (complete Freund adjuvant) is effected s.c. in the hind foot pad, near the tail and near the neck. After four and six weeks second and third administrations follow (incomplete Freund adjuvant) effected s.c. on the back and i.m. in the hind legs, respectively. Blood samples are collected one week after the third administration and the antisera titre and isotype distribution are analyzed by ELISA.

Those mice exhibiting highest titres and broadest isotype distribution are selected for further experimentation and given booster injections on days -4, -3, -2 and -1 prior to fusion using 50 microg of GM-CSF in 200 microl of 9% NaCl, by i.p. (50%) and i.v. (50%) injection on day -4, and by i.p. (100%) injection on days -3, -2 and -1.

Specifically, spleen cells of 3 mice with the isotype distribution $IgG_1/K$, $IgG_{2a}/K$ and $IgG_{2b}/K$ were selected.

## 2) Recovery of sensitized, antibody-producing spleen cells and fusion [steps 2. and 3.]

Recovery of sensitized, antibody-producing spleen cells and fusion are effected following published methods (S. Fazekas et al., J. Immunol. Methods 35 [1980] 1-32). Briefly, $2.5 \times 10^7$ or $5 \times 10^{-7}$ spleen cells from each mouse [obtained as described under 1)] are fused with $5 \times 10^7$ mouse (Balb/c) non-secreting myeloma cells using PEG 4000 and distributed into 24 x 24 wells and growing hybridomas selected in HAT medium.

Specifically, 1376 growing hybridomas out of 1764 seeded wells were obtained from the fusions.

## 3) Screening [step 4.]

Culture supernatants are screened in a direct binding ELISA for the presence of antibodies specifically recognizing primate GM-CSF as follows:
- ELISA plates are coated (3 hours; 37°) with CHO-expressed GM-CSF-Thr and E.coli-expressed GM-CSF-Thr in parallel (500 nanog/ml in phosphate-buffered saline [PBS]; unrelated protein (bovine serum albumin [BSA]) is used to determine non-specific binding.
- Microtiter plates saturation is performed by incubating BSA (2% in PBS; 2 hours, 37°); plates are washed, dried, sealed and kept at 4°.
- Hybridoma supernatants (diluted 1/11 in PBS-1% BSA) are incubated overnight at 4° in precoated plates.
- After washes, bound monoclonal antibodies are revealed by incubation first with rabbit Ig anti-mouse globulin and subsequently with goat Ig anti-rabbit globulin coupled to alkaline phosphatase (both at 1 microg/ml in PBS - 1% BSA; 2 hours, 37°).
- Hydrolysis of paranitrophenyl phosphate is measured at 405 nm.

Out of 797 supernatants analyzed for binding to solid-phase-coupled CHO-GM-CSF, 387 were specific for the immunizing antigen.

The fine specificity is determined using a parallel ELISA by comparing the result with CHO-expressed GM-CSF and E.coli-expressed GM-CSF to select those monoclonal antibodies (the vast majority) recognizing the protein moiety of GM-CSF.

Further, the culture supernatants are also submitted to isotype determination as follows: Indirect ELISA is performed as described above except that bound monoclonal antibodies are revealed using a set of mouse isotype- specific rabbit immunoglobulins, namely anti-gamma$_1$, -gamma$_{2a}$, -gamma$_{2b}$, -gamma$_3$, -mu, -kappa and -lambda (V. Quesniaux et al., Immunol. Lett. 9 [1985] 99-104).

Specifically, the isotype of 216 monoclonal antibodies was determined. Most were of the $IgG_1$-, a few were of the $IgG_{2a}$-, $IgG_{2b}$ - or $IgG_3$-isotype. Virtually all light chains were kappa.

Further, the physico-chemical properties of the monoclonal antibodies are also determined, namely, the relative affinity for GM-CSF, as follows: after a reaction has been obtained between the monoclonal antibody in soluble form and GM-CSF coupled to a solid surface, buffers of high molarity, abrupt pH changes or chaotropic ions are employed, in manner known for eluting proteins from affinity matrices, to challenge the stability of the binding. Using this system it is possible to establish a scale system to define the strength of the binding of the monoclonal antibody to GM-CSF. It is likely that the more or less pronounced readiness of the binding to be disrupted is related to the affinity of the monoclonal antibody to its antigen. This approach can be used to evaluate the binding stability of monoclonal antibodies.

From the screening effected as described above on nearly 800 supernatants three hybridoma cell lines were selected, secreting antibody to GM-CSF particularly indicated for use in affinity purification.

They all have:
- specificity for protein determinants of GM-CSF;
- reasonable affinity, i.e. no elution is observed with high salt concentration, and elution is possible under pH conditions not affecting the biological activity of GM-CSF; and
- growth requirements and production rates compatible with large-scale production.

Specifically, these three cell-lines produce monoclonal antibodies having the following characteristics:

## 1) First cell-line (5-2-4):
- Isotype: $IgG_{2b}$
- Physico-chemical properties: 20% antibody-GM-CSF binding dissociation at pH 4.5 in 0.1M acetate buffer
68% antibody-GM-CSF binding dissociation at pH 2.5 in 0.1M glycine buffer
70% antibody-GM-CSF binding dissociation at pH 6.3 in 3M NaSCN.

This cell-line, named 5-2-4, has been deposited on October 2, 1986 with the European Collection of Animal Cell Cultures (ECACC), Porton Down, Salisbury, Wiltshire SP4 0JG, United Kingdom, under Accession Number 86100202.

## 2) Second cell-line (1-113-4):
- Isotype: $IgG_1$
- Physico-chemical properties: 60% antibody-GM-CSF binding dissociation at pH 4.5 in 0.1M acetate buffer
62% antibody-GM-CSF binding dissociation at pH 2.5 in 0.M glycine buffer.

This cell-line, named 1-113-4, has also been deposited on October 2, 1986 with the ECACC, under Accession Number 86100203.

3) Third cell-line (L1-507-6):
- Isotype: IgG$_1$
- Physico-chemical properties: 7% antibody-GM-CSF binding dissociation at pH 4.5 in 0.1M acetate buffer
76% antibody-GM-CSF binding dissociation at pH 2.5 in glycine buffer
72% antibody-GM-CSF binding dissociation at pH 6.3 in 3M NaSCN
This cell-line, named 1-507-6, has also been deposited on October 2, 1986 with the ECACC, under Accession Number 86100204.

Example 2: Quantitative monitoring of GM-CSF

a) Preparation of a monoclonal antibody for use in quantitative monitoring of GM-CSF (fourth cell-line: 1-3-2)
Using the procedures described under Example 1 a hybridoma cell line secreting antibody to GM-CSF having the following characteristics was obtained:
- Isotype: IgG$_1$
- Physico-chemical properties: 49% antibody-GM-CSF binding dissociation at pH 7.2 in 0.5 NaCl
79% antibody GM-CSF binding dissociation at pH 4.5 in 0.1M acetate
64% antibody-GM-CSF binding dissociation at pH 2.5 in 0.1M glycine buffer
67% antibody-GM-CSF binding dissociation at pH 6.3 in 3M NaSCN.
This cell-line, named 1-3-2, has also been deposited on October 2, 1986 with the ECACC, under Accession Number 86100201.
It is apparent that this cell-line is particularly indicated for producing monoclonal antibodies for use in diagnostic/assay kits for GM-CSF, and in functional inactivation of GM-CSF.

b) Inhibition ELISA
CHO- as well as E.coli-expressed GM-CSF can be used. The sensitivity of the assay is in the nano-to picogram range. Thus 50% inhibition is obtained with about 0.4 to about 5 ng/ml GM-CSF, depending on assay conditions.
The method is as follows:
The binding of the monoclonal antibody (2.5 to 50 nanog/ml) to solid-phase-coupled GM-CSF (0.5 to 10 microg/ml) is inhibited by various concentrations of purified GM-CSF (0.01 to 40 nanog/ml) to obtain a standard calibration curve or by several dilutions of the sample solution with an unknown GM-CSF content. The detection system of this ELISA is as described under Example 1. The useful measurable range for GM-CSF is between 0.05 ng/ml and 20 ng/ml.
In a comparative test GM-CSF-transfected cells of 16 fermentation batches were extracted with sodium dodecyl sulfate and tested by both Western blot and ELISA:

Table

Determination of GM-CSF by Western blot and ELISA

| Sample | Western blot ca. % GM-CSF* | ELISA | |
|--------|---------------------------|-------|---|
| | | % GM-CSF* | microg GM-CSF/mg cell pellet |
| 1 | ∼ 100 | 100 | 3.5 |
| 2 | << 25 | 0.8 | 0.03 |
| 3 | ∼ 60 | 60 | 2.1 |
| 4 | << 25 | 0.8 | 0.03 |
| 5 | ∼ 50 | 42 | 1.5 |
| 6 | ∼ 30 | 39 | 1.4 |
| 7 | ∼ 30 | 21 | 0.7 |
| 8 | < 25 | 17 | 0.6 |
| 9 | ∼ 60 | 72 | 2.5 |
| 10 | ∼ 30 | 26 | 0.9 |
| 11 | ∼ 50 | 79 | 2.8 |
| 12 | ∼ 30 | 85 | 3.0 |
| 13 | ∼ 50 | 2 | 0.08 |
| 14 | << 25 | 0.3 | 0.01 |
| 15 | n.d. | 0.3 | 0.01 |
| 16 | n.d. | 27 | 1.0 |

* GM-CSF was calculated in relation to sample No. 1, which contains the highest amount of GM-CSF and was taken as 100%

n.d. = not determined

The sample containing the highest amount of GM-CSF was taken as 100% for the Western blot method (sample No. 1 in the Table) and the values for other samples were estimated in relation to this sample. From the Table it is seen that the amounts estimated by visualization of Western blot correspond well with the amounts determined by ELISA except for Samples 12 and 13. The advantages of ELISA over Western blot are:
- the method is less time consuming
- the expensive material (antibodies) is needed in small amounts
- it is more sensitive (the lowest amount still detectable is 0.05 ng GM-CSF/ml extract)
- it is a method which gives accurate spectrophotometrically measurable values, while the values obtained by Western blot are estimates only.

Example 3: GM-CSF affinity purification system

a) Preparation of a column for affinity chromatography of GM-CSF
The monoclonal antibodies of Example 1 are purified from culture supernatant (Iscove's medium, 1% foetal calf serum) by protein A chromatography to more than 95% purity as determined by high pressure liquid chromatography on hydroxyapathite and then coupled to CNBr-activated Sepharose ® 4B (3 mg monoclonal antibody to 1 ml gel, i.e. about 20 nanomoles).

b) Affinity chromatography of GM-CSF
Testing the affinity columns includes:
- binding and elution of purified GM-CSF to determine the recovery;
- application of supernatant of GM-CSF-transfected CHO cells to determine enrichment and capacity of the columns;
- determination of the biological activity of the affinity purified material
- determination of the purity by gel-chromatograhy.
The working temperature is 4°.
The following Table summarizes the results obtained with three different affinity columns:

## Table

| Antibody [1] | GM-CSF Source | Amount (microg) | Effluent [2] (microg) | Eluate [3] (microg) | Recovery (%) | Capacity (mole GM-CSF per mole antibody) |
|---|---|---|---|---|---|---|
| 5-2-4 | CHO | 50 [8] | 0.1 | 36 | 72 | – [7] |
|  | CHO | 1170 [4] | 574 | 570 | 96 | 1.2 |
|  | E.coli | 50 [5] | 0.4 | 23 | – [6] | – |
| 1-507-6 | CHO | 50 [8] | 1.5 | 28 | 56 | – |
| 1-113-4 | CHO | 50 [8] | 0.2 | 26 | 52 | – |

[1] 3 mg of monoclonal antibody of the type produced by the indicated hybridoma cell line, on 1 ml of CNBr-activated Sepharose ® 4B

[2] Phosphate-buffered saline (PBS)

[3] 0.1 M glycine/HCl buffer pH 2.5 with 0.14 M NaCl

[4] Cell-culture supernatant of GM-CSF-transfected CHO cells containing 6 microg/ml GM-CSF in 1% FCS medium as determined in ELISA

[5] partially purified

[6] Indeterminate since purity of starting material not determined

[7] An excess of GM-CSF supernatant is applied. 570 microg GM-CSF could be isolated with 3 mg antibody, which corresponds to 1.2 mole GM-CSF per mole antibody.

[8] Purified GM-CSF

Thus it is found that when using the column containing the monoclonal antibodies of line 5-2-4 more than 99% of CHO- and E.coli-GM-CSF is bound and can be eluted with glycine buffer pH 2.5. No elution is observed with acetate buffer pH 4.5. The recovery of GM-CSF is between 72% and 96%.

It also appears from the Table that similar results are obtained when using the columns containing the antibodies produced by cell-lines 1-507-6 and 1-113-4, the recoveries being 52% to 56% of the applied GM-CSF.

The CHO-GM-CSF affinity-purified from cell culture supernatant has been found to retain full biological activity when tested in the chronic myeloid leukemia assay (Griffin et al., Blood 63 (1984) 904-911). The purity was analyzed by sodium dodecyl sulfate-polyacrylamide gel electrophoresis. The affinity-purified material contained all sizes of GM-CSF with varying degrees of glycosylation (15-34 kD). Scanning of the affinity-purified GM-CSF revealed serum albumin as the only significant contaminant to amount to approximately 1%.

Example 4: Functional inactivation of GM-CSF-Thr

a) CML assay
A number of monoclonal antibodies were identified which neutralise GM-CSF in the CML bioassay. Of these 1-3-2 was the most effective.

Titration of antibody and GM-CSF concentrations demonstrated that 5 ng purified antibody from cell line 1-3-2 neutralises 1 unit GM-CSF, equivalent to about 0.03 ng protein.

The 5-2-4 monoclonal antibody was 2-5 times less effective in neutralising GM-CSF activity.

b) Human bone marrow precursor cells
GM-CSF promotes the differentiation and proliferation of precursor cells from normal human bone marrow. When bone marrow cells are seeded in a semisolid medium containing GM-CSF they form colonies of mature neutrophils, macrophages and eosinophils after 7-14 days of incubation. This biological activity of GM-CSF can be inhibited by addition of antibody from cell-line 1-3-2 at the beginning of the incubation period. The concentration of antibody is approximately 10 times that required in the CML assay.

**Claims**

1. A monoclonal antibody to primate GM-CSF.
2. An antibody according to claim 1 to human GM-CSF.
3. The antibody according to claim 1 which is produced by cell-line 1-3-2.
4. A hybridoma cell-line producing a monoclonal antibody to primate GM-CSF.
5. A cell-line according to claim 4 producing a monoclonal antibody to human GM-CSF.
6. The cell-line according to claim 4 which is 1-3-2.
7. A method for the production of a monoclonal antibody according to claim 1 which comprises recovering the monoclonal antibody produced by a hybridoma cell-line according to claim 4.
8. A method for the production of a hybridoma cell line according to claim 4 which comprises
    1. immunization by administration of an immunogenic GM-CSF preparation to an appropriate animal species;
    2. recovery of antibody-producing, e.g. spleen or lymph-node, cells sensitized to the antigen;
    3. immortalization of recovered cells, e.g. by fusion with an appropriate myeloma cell line, to produce hybridoma cell lines;
    4. selection of an immortalized cell clone, e.g. hybridoma line, producing monoclonal antibodies as required; and
    5. recovery of the monoclonal antibodies produced by the cell line.
9. A system for the large-scale affinity purification of primate GM-CSF comprising
- a support matrix and
- a monoclonal antibody according to claim 1.
10. An affinity purification system according to claim 9 wherein the monoclonal antibody is produced by cell-line 5-2-4, 1-113-4 or 1-507-6.
11. A system for quantitative monitoring of primate GM-CSF levels comprising a monoclonal antibody according to claim 1.
12. A system according to claim 11 which is an immunoassay kit or system for GM-CSF monitoring comprising
A) a monoclonal antibody according to claim 1 and
B) GM-CSF,
one of components A) or B) being in the form of a labelled derivative, appropriately together with further conventional components.
13. Use of a monoclonal antibody according to claim 1 in functionally inactivating primate GM-CSF.
14. Use according to claim 13 in therapy.

15. Use according to claim 13 in the prophylaxis or treatment of acute or chronic myeloid leukemia or other diseases involving autocrine production or overproduction of GM-CSF.

16. A monoclonal antibody according to claim 1 for use in the functional inactivation of primate GM-CSF.

17. A monoclonal antibody according to claim 16 for use in therapy.

18. A monoclonal antibody according to claim 16 for use in the prophylaxis or treatment of acute or chronic myeloid leukemia or other diseases involving autocrine production or overproduction of GM-CSF.

19. Substantially pure primate GM-CSF whenever prepared using a monoclonal antibody according to claim 1.

20. An immunological procedure wherein a monoclonal antibody according to claim 1 is used.

21. An immunological procedure according to claim 20 wherein the monoclonal antibody is produced by cell-line 1-3-2.

22. A process for the large-scale affinity purification of primate GM-CSF comprising affinity-chromatographing a GM-CSF-containing solution in a system as defined in claim 9.

23. Use of a system according to claim 9.

24. A pharmaceutical composition comprising a monoclonal antibody to primate GM-CSF.

25. A composition according to claim 24 wherein the monoclonal antibody is produced by cell-line 1-3-2.

Claims for the following contracting States: ES, GR:

1. A method for the production of a monoclonal antibody to primate GM-CSF which comprises recovering the monoclonal antibody produced by a hybridoma cell-line producing a monoclonal antibody to primate GM-CSF.

2. A method for the production of a hybridoma cell-line producing a monoclonal antibody to primate GM-CSF which comprises

    1. immunization by administration of an immunogenic GM-CSF preparation to an appropriate animal species;

    2. recovery of antibody-producing, e.g. spleen or lymph-node, cells sensitized to the antigen;

    3. immortalization of recovered cells, e.g. by fusion with an appropriate myeloma cell line, to produce hybridoma cell lines;

    4. selection of an immortalized cell clone, e.g. hybridoma line, producing monoclonal antibodies as required; and

    5. recovery of the monoclonal antibodies produced by the cell line.

3. A system for the large-scale affinity purification of primate GM-CSF comprising
- a support matrix and
- a monoclonal antibody to primate GM-CSF.

4. An affinity purification system according to claim 3 wherein the monoclonal antibody is produced by cell-line 5-2-4, 1-113-4 or 1-507-6.

5. A system for quantitative monitoring of primate GM-CSF levels comprising a monoclonal antibody to GM-CSF.

6. A system according to claim 5 which is an immunoassay kit or system for GM-CSF monitoring comprising
A) a monoclonal antibody to primate GM-CSF and
B) GM-CSF,
one of components A) or B) being in the form of a labelled derivative, appropriately together with further conventional components.

7. An immunological procedure wherein a monoclonal antibody to primate GM-CSF is used.

8. An immunological procedure according to claim 7 wherein the monoclonal antibody is produced by cell-line 1-3-2.

9. A process for the large-scale affinity purification of primate GM-CSF comprising affinity-chromatographing a GM-CSF-containing solution in a system as defined in claim 3.

10. Use of a system according to claim 3.